# EUROPEAN PATENT APPLICATION

(11) **EP 2 662 047 A1**
(43) Date of publication of application: **13.11.2013**
(21) Application number: 13167018.4
(22) Date of filing: 08.05.2013
(51) Int. Cl.: A61B 18/14

(54) **Apparatus for activating an electrosurgical vessel sealing instrument having an electrical cutting mechanism**

(30) Priority: 09.05.2012 US 201213467767
(71) Applicant: Covidien LP, Mansfield, MA 02048 (US)
(72) Inventor: Kerr, Duane E., Loveland, CO Colorado 80537 (US); Behnke, Robert J., Erie, CO Colorado 80516 (US); Waskiewicz, Alexander M., Boulder, CO Colorado 80301 (US); Romero, Paul R., Loveland, CO Colorado 80537 (US); Couture, Gary M., Longmont, CO Colorado 80501 (US)
(74) Representative: HOFFMANN EITLE

(57) **Abstract**

An electrosurgical forceps is provided and includes a housing, a shaft and a pair of opposing first and second jaw members. Each jaw member including a pair of spaced apart, electrically conductive tissue sealing surfaces adapted to connect to a source of electrosurgical energy such that the tissue sealing surfaces are capable of conducting electrosurgical energy through tissue held therebetween to effect a seal. One or both of the first and second jaw members includes an electrically conductive cutting element disposed thereon. A switch assembly operably disposed on the housing includes an activation member in operable communication with the source of electrosurgical energy for supplying electrosurgical energy to the first and second jaw members. Activation of the activation member provides electrosurgical energy to the tissue sealing surfaces for sealing tissue and electrosurgical energy to the cutting element for cutting the sealed tissue.

## Description

### BACKGROUND

### Technical Field

The present disclosure relates to apparatuses for activating an electrosurgical vessel sealing instrument having an electrical cutting mechanism. More particularly, the present disclosure relates to switching apparatuses utilized to activate a sealing and an electrode-cut sequence.

### Description of Related Art

Open or endoscopic electrosurgical forceps utilize both mechanical clamping action and electrical energy to effect hemostasis. Certain surgical procedures rely on a combination of clamping pressure, electrosurgical energy and gap distance to "seal" tissue, vessels and certain vascular bundles. More particularly, vessel sealing or tissue sealing utilizes a unique combination of radiofrequency energy, clamping pressure and precise control of gap distance (i.e., distance between opposing jaw members when closed about tissue) to effectively seal or fuse tissue between two opposing jaw members or sealing plates.

Typically, and particularly with respect to endoscopic electrosurgical procedures, once a vessel is sealed, the surgeon has to remove the sealing instrument from the operative site, substitute a new instrument through the cannula and accurately sever the vessel along the newly formed tissue seal. As can be appreciated, this additional step may be both time consuming (particularly when sealing a significant number of vessels) and may contribute to imprecise separation of the tissue along the sealing line due to the misalignment or misplacement of the severing instrument along the center of the tissue seal.

To overcome the aforementioned drawbacks, conventional sealing instruments sometimes utilize an end effector assembly that includes a cutting electrode configuration. In particular, the end effector may include jaw members that include respective electrically conductive vessel/tissue sealing surfaces, and an electrically conductive cutting element disposed within an insulator of one or both of the jaw members. The cutting element is capable of cutting vessels/tissue through electrosurgical activation. Typically, a potential of the electrically conductive sealing surfaces and the potential of the cutting elements are independently activatable by a surgeon through an interrelated combination of controls that include a handle, a trigger, a handswitch, and a footswitch. During use, a surgeon must activate these controls in a specific sequence to effectively seal and cut tissue. Often, several hand and/or finger manipulations are required to complete the desired sealing and cutting procedure.

### SUMMARY

Embodiments of the present disclosure are described in detail with reference to the drawing figures wherein like reference numerals identify similar or identical elements. As used herein, the term "distal" refers to the portion that is being described which is further from a user, while the term "proximal" refers to the portion that is being described which is closer to a user.

An aspect of the present disclosure provides an electrosurgical forceps. The electrosurgical forceps includes a housing supporting a shaft thereon. The shaft defines a longitudinal axis therethrough. A pair of opposing first and second jaw members each including a pair of spaced apart, electrically conductive tissue sealing surfaces that are adapted to connect to a source of electrosurgical energy. The tissue sealing surfaces are capable of conducting electrosurgical energy through tissue held therebetween to effect a seal. At least one of the first and second jaw members includes an electrically conductive cutting element disposed thereon. A switch assembly operably disposed on the housing includes an activation member in operable communication with the source of electrosurgical energy for supplying electrosurgical energy to the first and second jaw members. The activation member is movable from an inactive configuration to a first active configuration for providing electrosurgical energy to the tissue sealing surfaces for sealing tissue and a second active configuration for providing electrosurgical energy to the cutting element for cutting the sealed tissue.

The activation member may be operably disposed at a proximal end of the housing and may be movable along the longitudinal axis to place the activation member in one of the first and second active configurations. The activation member may be a depressible button that is depressed by differing amounts in the first and second active configurations. Alternatively, the activation member may be operably disposed adjacent a center of the housing and may be movable across the longitudinal axis to place the activation member in one of the first and second active configurations. The activation member may be accessible from left or right sides of the housing. In certain instances, the activation member may be one of a rocker switch and switch bar. In this instance, the rocker switch may include opposed, particularly top and bottom portions, that upon depression thereof are configured to place the rocker switch in respective first and second active configurations.

The switch bar may include top and bottom depressible portions that are configured to respectively place the switch bar in the first and second active configurations. The top and bottom depressible portions may be configured to illuminate when pressed. The switch bar may include left and right depressible portions that are configured to adjust a power intensity level of the electrosurgical energy provided to the first and second jaw members. In this instance, a plurality of horizontally placed illuminable members may be disposed between the left and right depressible portions to indicate to a user the power intensity level of the electrosurgical energy being provided to the first and second jaw members.

An aspect of the present disclosure provides an electrosurgical forceps. The electrosurgical forceps includes a housing and a pair of opposing first and second jaw members. At least one of the first and second jaw members is movable relative to the other from a first position wherein the first and second jaw members are disposed in spaced relation relative to one another to a second position wherein the first and second jaw members cooperate to grasp tissue therebetween. Each jaw member includes a pair of spaced apart, electrically conductive tissue sealing surfaces adapted to connect to a source of electrosurgical energy such that the tissue sealing surfaces are capable of conducting electrosurgical energy through tissue held therebetween to effect a seal. At least one of the first and second jaw members includes an electrically conductive cutting element disposed thereon. A first switch assembly operably disposed on the housing includes an activation member in operable communication with the source of electrosurgical energy for providing electrosurgical energy to the tissue sealing surfaces for sealing tissue. A lever having latched and unlatched configurations and in operable communication with a second switch assembly is in operable communication with the source of electrosurgical energy such that in the unlatched configuration electrosurgical energy is provided to the cutting element. A handle assembly includes a movable handle configured to move the at least one movable jaw member from the first position to the second position. The movable handle is in operable communication with the lever and configured to move the lever from the latched configuration to the unlatched configurations.

An aspect of the present disclosure provides an electrosurgical forceps. The electrosurgical forceps includes a housing and a pair of opposing first and second jaw members. Each jaw member includes a pair of spaced apart, electrically conductive tissue sealing surfaces surface adapted to connect to a source of electrosurgical energy such that the tissue sealing surfaces are capable of conducting electrosurgical energy through tissue held therebetween to effect a seal. At least one of the first and second jaw members includes an electrically conductive cutting element disposed thereon. A switch assembly includes an activation member and a selector. The activation member is in operable communication with the source of electrosurgical energy for providing electrosurgical energy to the first and second jaw members. The selector is configured to allow a user to selectively place the electrosurgical forceps in at least two modes of operation, a sealing mode and a cutting mode, wherein in the sealing mode, activation of the activation member supplies electrosurgical energy to the tissue sealing surfaces for sealing tissue and in the cutting mode, activation of the activation member supplies electrosurgical energy to the cutting element for cutting the sealed tissue.

The selector may be a rotary dial that is positioned on one of a left and right side of the housing. Or, the activation member may be a depressible button. The switch assembly including the activation member and the selector may be operably supported on the housing. The switch assembly including the selector may be operably supported on the housing and the activation member may be a footswitch. The selector may be configured to allow a user to selectively place the electrosurgical forceps in at least three modes of operation, a sealing mode, a cutting mode, and an auto mode, wherein in the auto mode of operation the cutting and sealing processes are automatically controlled by the source of electrosurgical energy.

An aspect of the present disclosure provides an electrosurgical forceps. The electrosurgical forceps includes a housing and a pair of opposing first and second jaw members. Each jaw member includes a pair of spaced apart, electrically conductive tissue sealing surfaces adapted to connect to a source of electrosurgical energy such that the tissue sealing surfaces are capable of conducting electrosurgical energy through tissue held therebetween to effect a seal. At least one of the first and second jaw members includes an electrically conductive cutting element disposed thereon. A switch assembly operably disposed on the housing includes a depressible activation member in operable communication with the source of electrosurgical energy for providing electrosurgical energy to the tissue sealing surfaces for sealing tissue. A footswitch is in operable communication with the source of electrosurgical energy for providing electrosurgical energy to the cutting element for cutting the sealed tissue. The footswitch may be functional only when the activation switch is depressed.

An aspect of the present disclosure provides an electrosurgical forceps. The electrosurgical forceps includes a housing and a pair of opposing first and second jaw members. Each jaw member includes a pair of spaced apart, electrically conductive tissue sealing surfaces adapted to connect to a source of electrosurgical energy such that the tissue sealing surfaces are capable of conducting electrosurgical energy through tissue held therebetween to effect a seal. At least one of the first and second jaw members includes an electrically conductive cutting element disposed thereon. A touchscreen operably disposed on the housing is in operable communication with at least one computer disposed in one of the housing and source of electrosurgical energy. The touchscreen provides at least two touchscreen buttons, a seal button and a cut button. Activation of the seal button provides electrosurgical energy to the tissue sealing surfaces to seal tissue and activation of the cut button provides electrosurgical energy to the cutting element to cut the sealed tissue. The touchscreen may be configured to provide feedback to a surgeon and may be configured to allow a surgeon to adjust a power intensity level of the electrosurgical energy provided to the tissue sealing surfaces cutting element.

An aspect of the present disclosure provides an electrosurgical forceps. The electrosurgical forceps includes a housing and a pair of opposing first and second jaw members. each jaw member including a pair of spaced apart, electrically conductive tissue sealing surfaces adapted to connect to a source of electrosurgical energy such that the tissue sealing surfaces are capable of conducting electrosurgical energy through tissue held therebetween to effect a seal. At least one of the first and second jaw members includes an electrically conductive cutting element disposed thereon. A switch assembly operably disposed on the housing includes an activation member in operable communication with the source of electrosurgical energy for providing electrosurgical energy to the tissue sealing surfaces for sealing tissue. A progressive touchpad operably disposed on the housing is in operable communication with at least one computer disposed in one of the housing and source of electrosurgical energy. The touchpad is configured to provide electrosurgical energy to the cutting element for cutting the sealed tissue upon activation thereof.

An aspect of the present disclosure provides an electrosurgical forceps. The electrosurgical forceps includes a housing supporting a rotatable shaft thereon. A pair of opposing first and second jaw members each includes a pair of spaced apart, electrically conductive tissue sealing surfaces adapted to connect to a source of electrosurgical energy. The tissue sealing surfaces are capable of conducting electrosurgical energy through tissue held therebetween to effect a seal, at least one of the first and second jaw members including an electrically conductive cutting element disposed thereon. A switch assembly is operably disposed on the housing and includes a magnetically-actuated activation member in operable communication with the source of electrosurgical energy for supplying electrosurgical energy to the first and second jaw members. The magnetically-actuated activation member includes a first magnet configured to close corresponding first switch when brought into contact therewith and a second magnet configured to close a corresponding second switch when brought into contact therewith. Activation of the first switch provides electrosurgical energy to the tissue sealing surfaces for sealing tissue and activation of the second switch active provides electrosurgical energy to the cutting element for cutting the sealed tissue.

The first and second magnets are supported in the housing and adjacent a proximal end of the shaft, and wherein movement of a slide on the housing brings the first and second magnets into contact with the corresponding switches.

An aspect of the present disclosure provides an electrosurgical forceps. The electrosurgical forceps includes a housing and a pair of opposing first and second jaw members. At least one of the first and second jaw members is movable relative to the other from a first position wherein the first and second jaw members are disposed in spaced relation relative to one another to a second position wherein the first and second jaw members cooperate to grasp tissue therebetween. Each jaw member includes a pair of spaced apart, electrically conductive tissue sealing surfaces adapted to connect to a source of electrosurgical energy such that the tissue sealing surfaces are capable of conducting electrosurgical energy through tissue held therebetween to effect a seal. At least one of the first and second jaw members includes an electrically conductive cutting element disposed thereon. At least one sensor is in operable communication with the source of electrosurgical energy and is configured to detect when the at least one moveable jaw member is in the second position and tissue is grasped between first and second jaw members. When the first and second jaw members are in the second position, the source of electrosurgical energy automatically provides electrosurgical energy to the tissue sealing surfaces for sealing tissue and, subsequently, provides electrosurgical energy to the cutting element for cutting the sealed tissue.

The at least one sensor may be in operable communication with a movable handle of the electrosurgical forceps and may be configured to detect when the movable handle has been moved into an unlatched configuration. The at least one sensor may be in operable communication with the first and second jaw members and may be configured to detect when the first and second jaw members are in the second position.

In the above described aspects and embodiments, the cutting element is fixed to the jaw member. The cutting element may be disposed laterally between the pair of sealing surfaces. The cutting element may protrude in a direction toward the other jaw member relative to the pair of sealing surfaces.

### BRIEF DESCRIPTION OF THE DRAWING

Various embodiments of the present disclosure are described hereinbelow with references to the drawings, wherein:

Fig. 1A is a right, perspective view of an endoscopic bipolar forceps having a switching apparatus according to an embodiment of the present disclosure;

Fig. 1B is an enlarged, schematic end view showing an electrode assembly configured for use with the endoscopic bipolar forceps depicted in Fig. 1A;

Fig. 2A is an enlarged, schematic view of the area of detail of Fig. 1;

Figs. 2B-2C are enlarged, elevational views of the switching apparatus depicted in Fig. 1 in various configurations;

Fig. 3A is schematic view of a housing of an endoscopic bipolar forceps having a switching apparatus according to yet another embodiment of the present disclosure;

Fig. 3B is a bottom view of the housing depicted in Fig. 3A;

Fig. 4 is schematic view of a housing of an endoscopic bipolar forceps having a switching apparatus according to still yet another embodiment of the present disclosure;

Fig. 5 is schematic view of a switching apparatus according to still yet another embodiment of the present disclosure;

Fig. 6 is schematic view of a housing of an endoscopic bipolar forceps having a switching apparatus according to an embodiment of the present disclosure;

Fig. 7 is schematic view of a housing of an endoscopic bipolar forceps having a switching apparatus according to still another embodiment of the present disclosure;

Figs. 8A and 8B are schematic views of a housing of an endoscopic bipolar forceps having a switching apparatus according to still yet another embodiment of the present disclosure, wherein Fig. 8A illustrates a lever of the switching apparatus in a latched configuration and Fig. 8B illustrates the lever in an unlatched configuration;

Fig. 9 is schematic view of a housing of an endoscopic bipolar forceps having a switching apparatus according to still yet another embodiment of the present disclosure;

Fig. 10 is schematic view of a housing of an endoscopic bipolar forceps having a switching apparatus according to still yet another embodiment of the present disclosure;

Fig. 11 is schematic view of a housing of an endoscopic bipolar forceps having a switching apparatus according to still yet another embodiment of the present disclosure;

Fig. 12A is schematic view of a housing of a switching apparatus according to still yet another embodiment of the present disclosure; and

Fig. 12B is a cross-sectional view taken along line segment 12B-12B in Fig. 12A.

### DETAILED DESCRIPTION

Detailed embodiments of the present disclosure are disclosed herein; however, the disclosed embodiments are merely examples of the disclosure, which may be embodied in various forms. Therefore, specific structural and functional details disclosed herein are not to be interpreted as limiting, but merely as a basis for the claims and as a representative basis for teaching one skilled in the art to variously employ the present disclosure in virtually any appropriately detailed structure. In this description, as well as in the drawings, like-referenced numbers represent elements which may perform the same, similar, or equivalent functions.

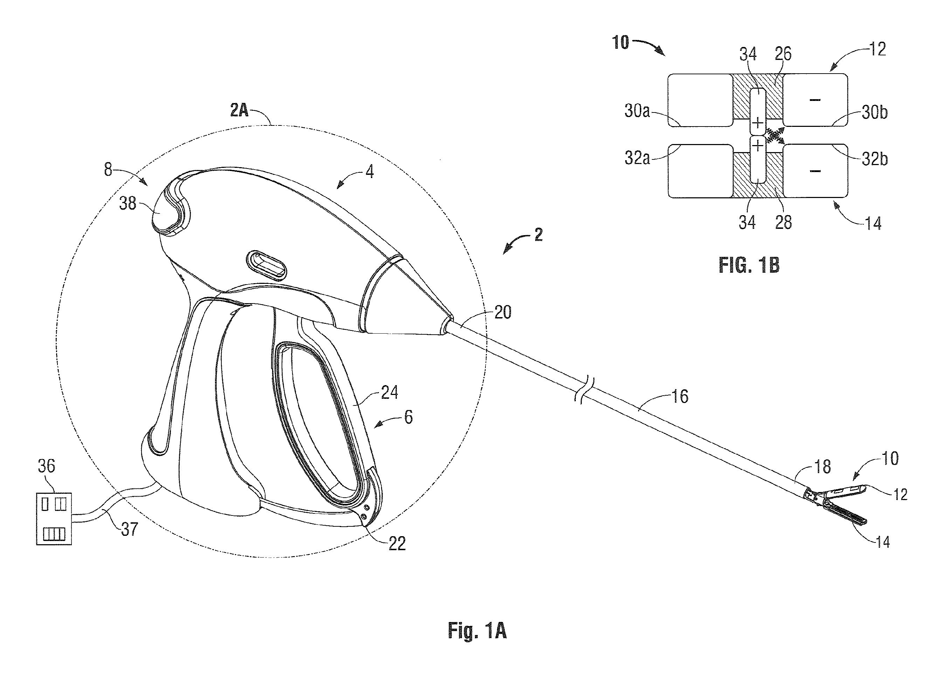

Turning now to Fig. 1A, a bipolar forceps 2 for use with various endoscopic surgical procedures generally includes a housing 4, a handle assembly 6, an activation switch assembly 8 and an electrode assembly 10 having opposing jaw members 12 and 14 which mutually cooperate to grasp, seal and divide tubular vessels and vascular tissue. Forceps 2 includes a shaft 16 which has a distal end 18 dimensioned to mechanically engage the electrode assembly 10 and a proximal end 20 which mechanically engages the housing 4. Shaft 16 defines a longitudinal axis "A-A" therethrough and may include one or more known mechanically engaging components which are designed to securely receive and engage the electrode assembly 10 such that the one or both of the jaw members 12 and 14 is/are pivotable relative to one another to engage and grasp tissue therebetween. While the illustrated embodiment depicts electrode assembly 10 having a unilateral jaw configuration, a bilateral jaw configuration may also be utilized.

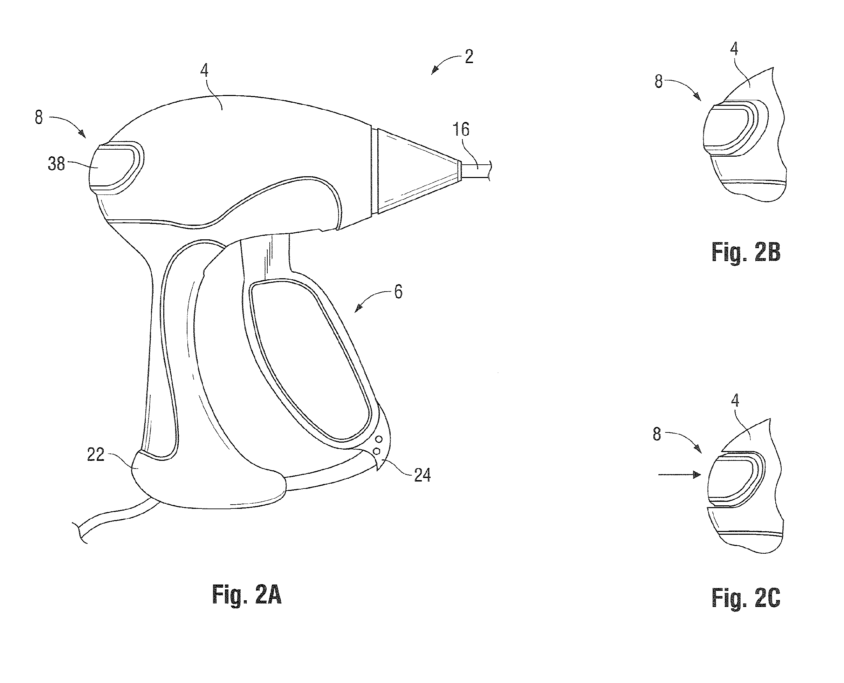

Handle assembly 6 includes a fixed handle 22 and a movable handle 24 (Figs. 1A and 2A) Fixed handle 22 is integrally associated with housing 4 and movable handle 24 is movable relative to fixed handle 22 to actuate the opposing jaw members 12 and 14 of the electrode assembly 10. In embodiments, movable handle 24 may be movable a predetermined distance proximally to "latch" the movable handle 24. In this instance, one or more components (e.g., relays, solenoids, latching devices, gears, links, combinations thereof, etc.) may operably couple to the movable handle 24 (or the fixed handle 22) to latch and unlatch the movable handle 24.

Continuing with reference to Fig. 1A, and with reference to Figs. 2A-2C, a switch assembly 6 is illustrated. Switch assembly 8 is operably coupled to a proximal end of the housing 4 and includes circuitry that allows the switch assembly 8 to communicate with a generator 36 that couples to the forceps 2 via a cable 37 (Fig. 1A). Switch assembly 8 includes an activation member 38 that is moveable between one or more positions. In particular, activation member 38 is moveable from an inactive configuration (Fig. 2A) to one or more active configurations (Figs. 2B-2C). In the inactive configuration, electrosurgical energy is not supplied to the jaw members 12 and 14. In the active configurations, electrosurgical energy is supplied to the jaw members 12 and 14. In particular, in a first active configuration (see Fig. 2B for example), electrosurgical energy is supplied to the jaw members 12 and 14 to seal tissue grasped therebetween. In a second active configuration, (see Fig. 2C for example), electrosurgical energy is supplied to the jaw members 12 and 14 to cut tissue that has been previously sealed. A tactile feedback mechanism (not shown) may be provided with the switch assembly 6 to indicate to a user when the activation member 38 has been placed in either of the first or second active configurations.

Referring now to Fig. 1B, electrode assembly 10 is illustrated including jaw members 12 and 14. An insulator or insulative material 26 and 28 is respectively disposed between each pair of electrically conductive sealing surfaces 30a, 30b and 32a, 32b on jaw members 12 and 14. Insulators 26, 28 may be made from any suitable non-conductive material. In the illustrated embodiment, insulators 26, 28 are made from ceramic material due to its hardness and inherent ability to withstand high temperature fluctuations,

One or both of the jaw members 12 and 14 includes an electrically conductive cutting element 34 disposed substantially within or disposed on the insulators 26, 28. In the illustrated embodiment each jaw member 12 and 14 includes a cutting element 34. In certain instances, one or both of the cutting elements 34 may be made from an insulative material with a conductive coating disposed thereon or one of the cutting elements 34 may be non-conductive.

Cutting elements 34 may be independently activated by a surgeon (or automatically activated by generator 36 once sealing is complete; this embodiment is described in greater detail below). When the activation member 38 is placed in the second active configuration (Fig. 2C), electrosurgical energy is supplied to one or both of the cutting elements 34 to cut tissue that has been sealed.

An audible or visual indicator (not shown) may be employed to indicate to a surgeon that the forceps 2 is ready for a sealing or cutting phase of the surgical procedure. For example, and in one particular embodiment, the generator 36 may be configured to provide an audible tone to the surgeon that is unique to a sealing phase. Likewise, the generator 36 may be configured to provide an audible tone to the surgeon that is unique to a cutting phase. Alternately, the forceps 2 may be configured to provide one of the aforementioned audible tones to the surgeon when the activation member 38 is placed either of the first or second active configurations.

A more detailed description of the operative features of the jaw members 12 and 14 including cutting elements 34 and/or seal surfaces 30a, 30b and 32a, 32b is discussed in commonly-owned U.S. Patent No. 7,276,068 filed on September 2, 2004 by Johnson et al.

In one particular embodiment, a safety algorithm may be employed to assure that an accurate and complete tissue seal is formed before cutting. For example, an audible or visual indicator may be employed to assure the surgeon that an accurate seal has been formed. In this instance, a surgeon may be prevented from moving the activation member 38 from the first active configuration to the second active configuration until the audible or visual indicator is communicated to the surgeon. In this instance, one or more locking features may be coupled to the switch assembly 6 and/or activation member 38.

In use, to seal tissue, activation member 38 is placed in the first active configuration (Fig. 2B) and the opposing sealing surfaces 30a, 32a and 30b, 32b are activated to seal the tissue disposed therebetween to create two tissue seals on either side of the insulators 26 and 28.

To cut tissue, activation member 38 is placed in the second active configuration (Fig. 2C) and the cutting elements 34 are energized with a first electrical potential "+" and the right opposing sealing surfaces 30b and 32b are energized with a second electrical potential "-" (Fig. 1B). This creates a concentrated electrical path between the potentials "+" and "-" through the tissue to cut the tissue between the previously formed tissue seals. Once the tissue is cut, the jaw members 12 and 14 are opened to release the two tissue halves.

As can be appreciated, the aforementioned switch assembly 8 allows a surgeon to initiate a sealing and cutting sequence without the need of several hand and/or finger manipulations as is typically required with conventional electrosurgical instruments.

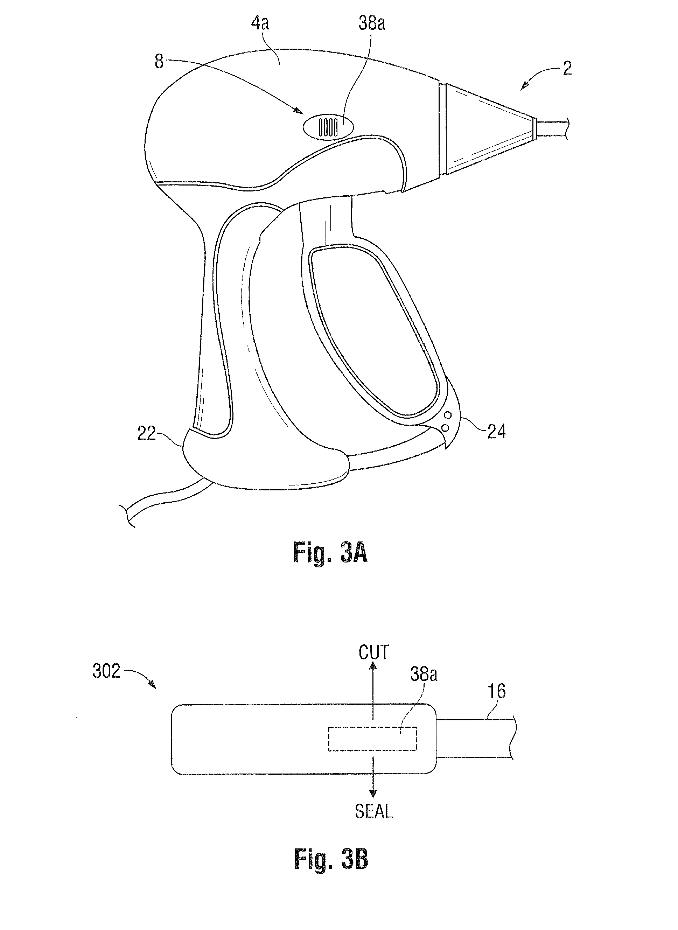

Switch assembly 8 may include various other types of activation members. For example, Figs. 3A and 3B illustrate an activation member 38a that is operably disposed adjacent a center portion 4a of the housing 4. In this instance, the activation member 38a is movable across the longitudinal axis "A-A" to place the activation member 38a in either the first or second active configurations, see Fig. 3B for example where the activation member 38a is shown in phantom. Activation member 38a is accessible from either the left or right sides of the housing 4 (see Fig. 3A); this allows a surgeon to depress the activation member 38a with one of the five digits from either the left and/or right sides. In one particular embodiment, the activation member 38a is movable across the longitudinal axis "A-A" when the movable handle 24 is in a latched configuration; this serves as a safety mechanism to prevent cutting sealed tissue when the jaws 12 and 14 are in an open configuration. That is, in the latched configuration the jaw members 12 and 14 are maintained in a closed configuration.

In use, and in one particular embodiment, activation member 38a is configured such that movement thereof to the left of the longitudinal axis "A-A" places the activation member 38a in the first active configuration and movement thereof to the right of longitudinal axis places the activation member in the second active configuration, or vice versa.

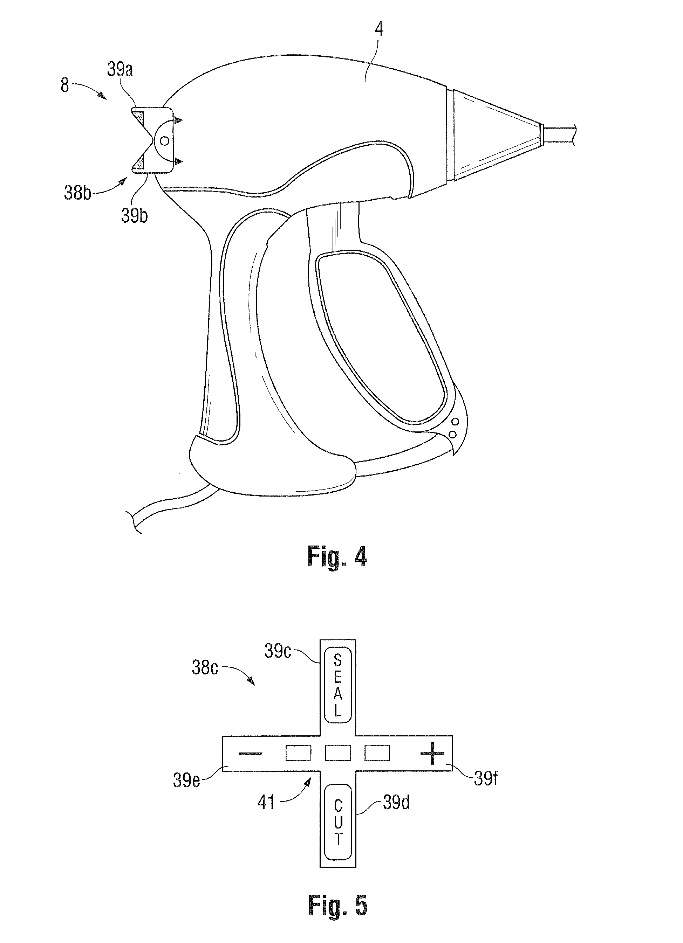

Fig. 4 illustrates an activation member that is in the form of a rocker switch 38b that is disposed vertically with respect to the longitudinal axis "A-A." In this instance, depressing a top portion 39a of the rocker switch 38b places the rocker switch 38b in the first active configuration and depressing a bottom portion 39b of the rocker switch 38b places the rocker switch 38b in the second active configuration, or vice versa. Alternately, the rocker switch 38b may be disposed horizontally with respect to the longitudinal axis "A-A." In this instance, the top and bottom portions 39a and 39b are replaced by left and ride side portions (not shown). The exact configuration of the rocker switch 38 will depend on a manufacturer's preference, surgical procedure, etc.

Fig. 5 illustrates an activation member that is in the form of a switch bar 38c that includes top and bottom depressible portions 39c and 39d, respectively, that are configured to respectively place the switch bar 38c in the first and second active configurations. In the embodiment illustrated in Fig. 5, the top and bottom depressible portions 39c and 39d are configured to illuminate when pressed. To this end, one or more types of devices, e.g., LEDs, backlighting, etc., may be operably coupled to the switch bar 38c to illuminate the top and bottom depressible portions 39c and 39d when they are pressed.

In the embodiment illustrated in Fig. 5, the switch bar 38c includes left and right depressible portions 39e and 39f, respectively, that are configured to adjust a power intensity level of the electrosurgical energy provided to the jaw members 12 and 14. In this particular instance, a plurality of horizontally placed illuminable members 41 may be disposed between the left and right depressible portions 39e and 39f to indicate to a surgeon the power intensity level of the electrosurgical energy that is being provided to the first and second jaw members 12 and 14.

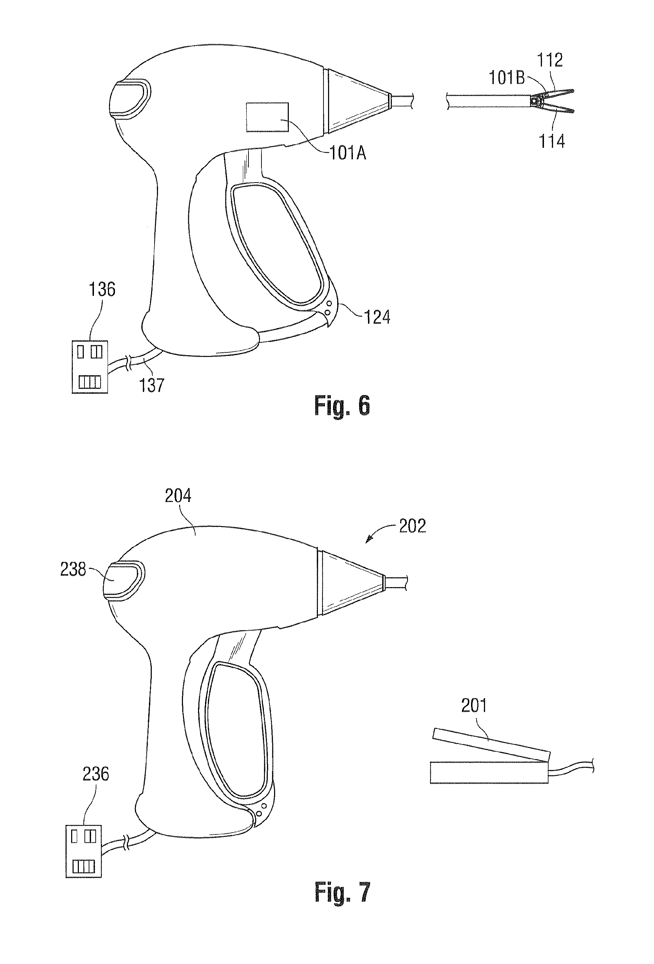

With reference to Fig. 6, a forceps 102 according to an alternate embodiment of the present disclosure is illustrated. Only those features unique to forceps 102 are described in detail. Unlike forceps 2 that utilizes a switch assembly 8, a generator 136 is configured to automatically detect when tissue is clamped between the jaw members 112 and 114 and ready for sealing and/or cutting. In this instance, one or more sensors 101a may be in operable communication with jaw members 112 and 114 and/or the movable handle 124 (Fig. 6). For example, and in one particular embodiment, sensor 101a may be configured to detect when the movable handle 124 has been moved into a "latched" configuration. In this instance, sensor 101a may be in operable communication with one or more components of the generator 136 and may be configured to send a command signal thereto that indicates that tissue is ready for sealing. Detection of this command signal by the generator 136 automatically initiates a sealing phase. Electrosurgical energy is then transmitted to pairs of tissue sealing surfaces 130a, 132a and 130b, 132b of respective jaw members 112 and 114 to seal tissue according to a specific predetermined duty cycle.

Subsequently, the generator 136 may provide electrosurgical energy to the cutting elements 134 to cut the sealed tissue. The cutting phase may be initiated as a result of command signal provided by a second sensor 101b that is configured to detect a seal quality of sealed tissue. In this particular instance, second sensor 101b may be positioned adjacent the jaw members 112 and 114 and configured to detect one or more tissue parameters associated therewith, e.g., information pertaining to tissue impedance, tissue temperature, etc., and communicate this information to one or more components of the generator 136. In the instance where the generator 136 determines that the seal quality is adequate, the generator 136 provides electrosurgical energy to the cutting elements 134 to cut the sealed tissue.

Alternately, the generator 136 may be configured to provide electrosurgical energy to the cutting elements 134 for cutting the sealed tissue subsequent to the end of the duty cycle. In this instance, the generator 136 may provide electrosurgical energy to the cutting elements 134 based on a specific predetermined cutting duty cycle.

In embodiments, the sealing phase and/or cutting phase may be initiated as a result of tissue being positioned between the jaw members 112 and 114 and the jaw members 112 and 114 being in a clamping position. In this instance, sensor 101b is configured to send a command signal that indicates to the generator 136 that tissue is ready for sealing and/or cutting.

As can be appreciated, the unique configuration of the forceps 102 including the sensors 101a and 101b in communication with the generator 136 provides a "hands-free" alternative for sealing and electrosurgically cutting tissue when compared to conventional forceps that are configured to seal and electrosurgically cut tissue.

With reference to Fig. 7, a forceps 202 according to an alternate embodiment of the present disclosure is illustrated. Only those features unique to forceps 202 are described in detail. A switch assembly 208 is operably disposed on the housing 204 and includes a depressible activation member 238 in operable communication with the generator 236 for providing electrosurgical energy to the tissue sealing surfaces 230a, 232a and 230b, 232b for sealing tissue. Unlike the activation member 38, however, activation member 238 is movable from an initial configuration to a single, active configuration.

A footswitch 201 is in operable communication with the generator 236 for providing electrosurgical energy to the cutting elements 234 for cutting the sealed tissue. In particular, after tissue is sealed, a surgeon activates, e.g., depresses, the footswitch 201 to cut the sealed tissue. The footswitch 201 can be configured such that activation thereof signals the generator 236 to initiate a cutting phase having a predetermined cutting duty cycle. In this instance, the generator 236 provides electrosurgical energy to the cutting elements 234 for the duration of the cutting duty cycle. The footswitch 201 may be configured to interrupt or stop the cutting duty cycle. For example, a surgeon may depress the footswitch 201 during the cutting duty cycle. Those skilled in the art will appreciate that the footswitch 234 can be configured to function in a variety of different manners. For example, and in one particular embodiment, the generator 234 provides electrosurgical energy to the cutting element 234 when both the footswitch 234 and the activation member 238 are in active configurations.

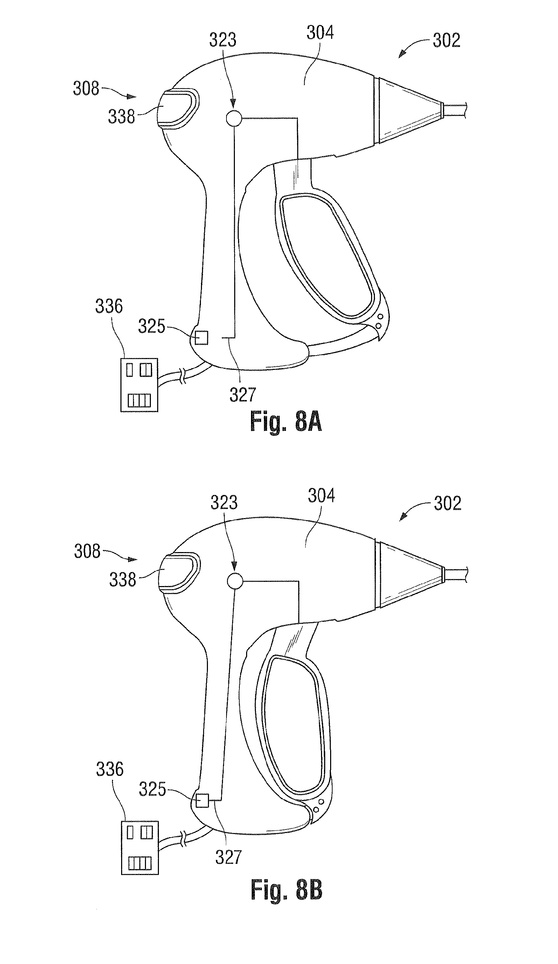

With reference to Figs. 8A and 8B, a forceps 302 according to an alternate embodiment of the present disclosure is illustrated. Only those features unique to forceps 302 are described in detail. A lever assembly 323 is operably disposed in the housing 304 and is in operable communication with the generator 336 via a second switch assembly 325. Lever 323 is movable from a latched configuration (Fig. 8A) to an unlatched configuration (Fig. 8B). In the unlatched configuration, a bottom portion 327 of the lever 323 contacts the switch 325, which, in turn, provides electrosurgical energy to the cutting elements 334. Electrosurgical energy may be provided to the cutting elements 334 in accordance with a cutting duty cycle and may be interrupted as a result of the lever 323 moving out of contact with the switch 325.

Movable handle 324 of the handle assembly 306 is configured to move jaw member 310 from an open configuration to a closed configuration. In addition, movable handle 324 is configured to move the lever 323 from the latched configuration (which corresponds to the jaw members being in the open configuration, see Fig. 1A for example) to the unlatched configurations (which corresponds to the jaw members being in the closed configuration not explicitly shown) as a result of the movable handle 324 being moved a predetermined distance proximally.

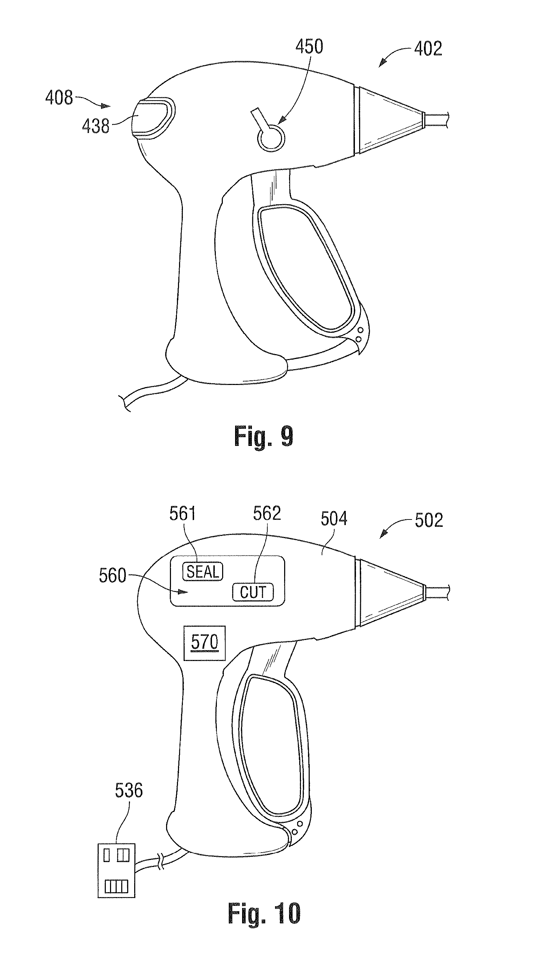

With reference to Fig. 9, a forceps 402 according to an alternate embodiment of the present disclosure is illustrated. Only those features unique to forceps 402 are described in detail. Switch assembly 408 includes activation member 438 and a selector 450. The selector 450 is configured to allow a surgeon to selectively place the forceps 402 in one or more modes of operation, a sealing mode, a cutting mode and an optional auto mode. Sealing mode, cutting mode and the optional auto mode are carried out in a manner as described above (see e.g., above with respect to the embodiments illustrated in Figs. 2A-2C and Fig. 6).

Selector 450 may be any suitable type of selector including, but not limited to slides, buttons, protrusions and the like. For illustrative purposes, the selector is shown as a rotary dial that may be positioned on either a left or right side of housing. In the embodiment illustrated in Fig. 9, the switch assembly 408 including the activation member 438 and the selector 450 are operably supported on the housing 404. In certain embodiments, the switch assembly 408 including the selector 450 may be operably supported on the housing 404 and a footswitch 401 may be utilized in manner as described above, see discussion with respect to the embodiment illustrated in Fig. 7. Or, in certain instances, the switch assembly 408 may be eliminated and the functions provided therefrom may provided by the footswitch 401.

With reference to Fig. 10, a forceps 502 according to an alternate embodiment of the present disclosure is illustrated. Only those features unique to forceps 502 are described in detail. A touchscreen 560 is operably disposed on the housing 504 and is in operable communication one or more computers 570. One computer 570 is illustrated in the drawings. Computer 570 may be disposed in either the housing 504 or generator 536. For illustrative purposes, the computer 570 is shown disposed in the housing 504. The computer 570 is configured to process information provided by the touchscreen 560 and communicates this information to one or more components associated with the generator 534.

Touchscreen 560 includes one or more touchscreen buttons. In the embodiment illustrated in Fig. 10, touchscreen 560 includes a touchscreen seal button 561 and a touchscreen cut button 562 (an optional touchscreen auto button may also be provided). To start a sealing phase, a surgeon presses, via a finger (or passive object, e.g., a stylus), the touchscreen seal button 561 and to start a cutting phase, a surgeon presses the touchscreen cut button 562. Electrosurgical energy is provided to the forceps 502 to seal and cut tissue in a manner as described hereinabove.

The touchscreen 560 may be configured to display information to a surgeon during either the sealing phase or cutting phase. Information that the touchscreen 560 displays may include, but is not limited to, date and time, seal time, cut time, time remaining in either of the seal or duty cycles, tissue parameters, power output, etc.

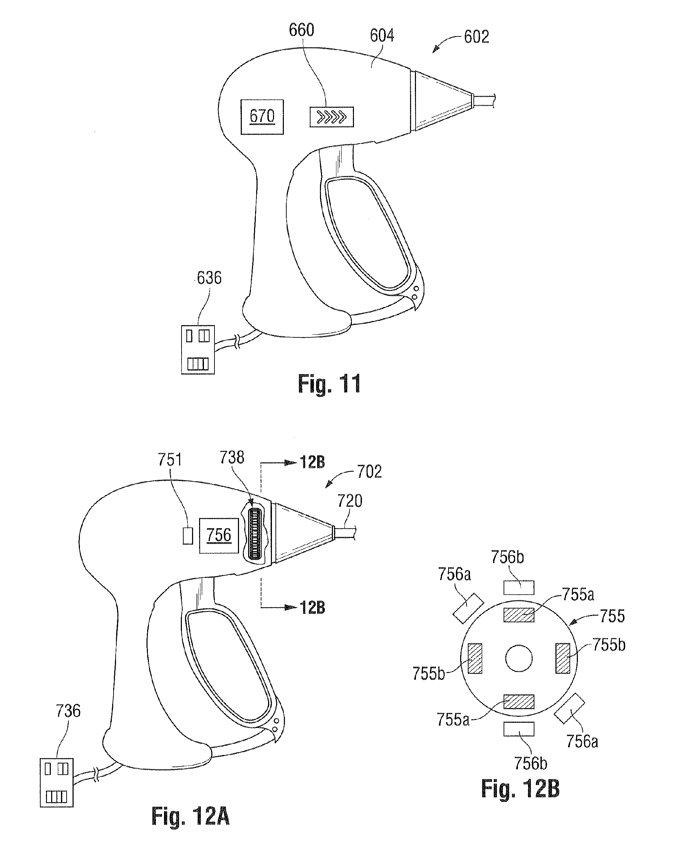

With reference to Fig. 11, a forceps 602 according to an alternate embodiment of the present disclosure is illustrated. Only those features unique to forceps 602 are described in detail. A progressive touchpad 660 is in operable communication one or more computers 670. One computer 670 is illustrated in the drawings. Computer 670 may be disposed in either the housing 604 or generator 636. For illustrative purposes, the computer 670 is shown disposed in the housing 604. The computer 670 is configured to process information provided by the touchpad 660 and communicates this information to one or more components associated with the generator 634. To activate a cutting phase, a surgeon runs his/her finger (or passive object, e.g., a stylus) across the screen of the touchpad 660.

With reference to Figs. 12A and 12B, a forceps 702 according to an alternate embodiment of the present disclosure is illustrated. Only those features unique to forceps 702 are described in detail. A switch assembly 708 is operably disposed on the housing 704 and includes a magnetically-actuated activation member 738 that communicates with a corresponding switch 756 disposed in operable communication with generator 736 for supplying electrosurgical energy as described hereinbefore (Fig. 12A). The magnetically-actuated activation member 738 is provided in the housing 704 and positioned adjacent the proximal end 720 of the shaft 716 and rotatably coupled thereto.

Magnetically-actuated activation member 738 includes one or more magnets 755 (Fig. 12B) that are configured to open and close a corresponding switch 756 (Fig. 12A). In the embodiment illustrated in Figs. 12A and 12B, the magnetically-actuated activation member 738 includes a pair of first magnets 755a configured to close corresponding first switches 756a and a pair of second magnets 755b configured to close corresponding second switches 756b (Fig. 12B). In particular, when the magnets 755a and 755b are brought into contact (or in close proximity) with the corresponding switches 756a and 756b, the switches 756a and 756b provide a command signal to the generator 736. More particularly, when the magnets 755a come into contact (or in close proximity) with the first switches 756a, electrosurgical energy is provided to seal tissue. In one particular embodiment, turning the magnetically-actuated activation member 738 a ¼ turn to the left from its initial configuration (Fig. 12B) places the magnets 755a into contact (or close proximity) with the first switches 756a and electrosurgical energy is provided to seal tissue. Likewise, when the magnets 755b come into contact (or in close proximity) with the first switches 756b, electrosurgical energy is provided to cut tissue. In one particular embodiment, turning the magnetically-actuated activation member 738 a ½ turn to the right from its initial configuration places the magnets 755b into contact (or in close proximity) with the second switches 756b and electrosurgical energy is provided to cut tissue.

A slide 751 (or other suitable device) may be utilized to move the magnetically-actuated activation member 738 between the ¼ and ½ turns. In this instance, moving the slide 571 to the left moves the magnetically-actuated activation member 738 a ¼ turn to the left from its initial configuration and moving the slide 571 to the right moves the magnetically-actuated activation member 738 a ½ turn to the right from its initial configuration.

Alternately, the magnetically-actuated activation member 738 may be fixedly supported on the shaft 716 such that rotation of the shaft 716 brings the first and second magnets 755a and 755b into contact (or close proximity) with the corresponding switches 756a and 756b.

From the foregoing and with reference to the various figure drawings, those skilled in the art will appreciate that certain modifications can also be made to the present disclosure without departing from the scope of the same. For example, while the aforementioned switch assemblies have been described herein as being an operational component of the forceps 2, it is within the purview of the present disclosure to provide one or more of the switch assemblies may be configured as a modular component that may be removably coupleable to the forceps 2.

In addition, either the forceps 2 or the generator 36 may include wireless technology to communicate with a surgeon such that the surgeon may switch between a sealing phase and a cutting phase when tissue is grasped between the jaw members 12 and 14. In this instance, "Bluetooth" technology, for example, may be included in either the forceps 2 or generator 36.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

### Items of the Disclosure

1. An electrosurgical forceps, comprising:
   a housing;
   a pair of opposing first and second jaw members, at least one of the first and second jaw members movable relative to the other from a first position wherein the first and second jaw members are disposed in spaced relation relative to one another to a second position wherein the first and second jaw members cooperate to grasp tissue therebetween, each jaw member including a pair of spaced apart, electrically conductive tissue sealing surfaces adapted to connect to a source of electrosurgical energy such that the tissue sealing surfaces are capable of conducting electrosurgical energy through tissue held therebetween to effect a seal, at least one of the first and second jaw members including an electrically conductive cutting element disposed thereon;
   a first switch assembly operably disposed on the housing and including an activation member in operable communication with the source of electrosurgical energy for providing electrosurgical energy to the tissue sealing surfaces for sealing tissue;
   a lever having latched and unlatched configurations and in operable communication with a second switch assembly in operable communication with the source of electrosurgical energy such that in the unlatched configuration electrosurgical energy is provided to the cutting element; and
   a handle assembly including a movable handle configured to move the at least one movable jaw member from the first position to the second position, the movable handle in operable communication with the lever and configured to move the lever from the latched configuration to the unlatched configurations.
2. An electrosurgical forceps, comprising:
   a housing;
   a pair of opposing first and second jaw members, each jaw member including a pair of spaced apart, electrically conductive tissue sealing surfaces surface adapted to connect to a source of electrosurgical energy such that the tissue sealing surfaces are capable of conducting electrosurgical energy through tissue held therebetween to effect a seal, at least one of the first and second jaw members including an electrically conductive cutting element disposed thereon; and
   a switch assembly including an activation member and a selector, the activation member in operable communication with the source of electrosurgical energy for providing electrosurgical energy to the first and second jaw members and the selector configured to allow a user to selectively place the electrosurgical forceps in at least two modes of operation, a sealing mode and a cutting mode, wherein in the sealing mode, activation of the activation member supplies electrosurgical energy to the tissue sealing surfaces for sealing tissue and in the cutting mode, activation of the activation member supplies electrosurgical energy to the cutting element for cutting the sealed tissue.
3. An electrosurgical forceps according to item 2, wherein the selector is a rotary dial, optionally that is positioned on one of a left and right side of the housing.
4. An electrosurgical forceps according to item 2 or 3, wherein the activation member is a depressible button.
5. An electrosurgical forceps according to item 2, 3 or 4, wherein the switch assembly including the activation member and the selector are operably supported on the housing.
6. An electrosurgical forceps according to item 2 or 3, wherein the switch assembly including the selector are operably supported on the housing and the activation member is a footswitch.
7. An electrosurgical forceps according to any one of items 2 to 6, wherein the selector is configured to allow a user to selectively place the electrosurgical forceps in at least three modes of operation, a sealing mode, a cutting mode, and an auto mode, wherein in the auto mode of operation the cutting and sealing processes are automatically controlled by the source of electrosurgical energy.
8. An electrosurgical forceps, comprising:
   a housing;
   a pair of opposing first and second jaw members, each jaw member including a pair of spaced apart, electrically conductive tissue sealing surfaces adapted to connect to a source of electrosurgical energy such that the tissue sealing surfaces are capable of conducting electrosurgical energy through tissue held therebetween to effect a seal, at least one of the first and second jaw members including an electrically conductive cutting element disposed thereon;
   a switch assembly operably disposed on the housing and including a depressible activation member in operable communication with the source of electrosurgical energy for providing electrosurgical energy to the tissue sealing surfaces for sealing tissue; and
   a footswitch in operable communication with the source of electrosurgical energy for providing electrosurgical energy to the cutting element for cutting the sealed tissue.
9. An electrosurgical forceps according to item 8, wherein the footswitch is functional only when the activation switch is depressed.
10. An electrosurgical forceps, comprising:
   a housing;
   a pair of opposing first and second jaw members, each jaw member including a pair of spaced apart, electrically conductive tissue sealing surfaces adapted to connect to a source of electrosurgical energy such that the tissue sealing surfaces are capable of conducting electrosurgical energy through tissue held therebetween to effect a seal, at least one of the first and second jaw members including an electrically conductive cutting element disposed thereon; and
   a touchscreen operably disposed on the housing and in operable communication with at least one computer disposed in one of the housing and source of electrosurgical energy, the touchscreen providing at least two touchscreen buttons, a seal button and a cut button,
   wherein activation of the seal button provides electrosurgical energy to the tissue sealing surfaces to seal tissue and activation of the cut button provides electrosurgical energy to the cutting element to cut the sealed tissue.
11. An electrosurgical forceps according to item 10, wherein the touchscreen is configured to provide feedback to a surgeon and/or is configured to allow a surgeon to adjust a power intensity level of the electrosurgical energy provided to the tissue sealing surfaces cutting element.
12. An electrosurgical forceps, comprising:
   a housing;
   a pair of opposing first and second jaw members, each jaw member including a pair of spaced apart, electrically conductive tissue sealing surfaces adapted to connect to a source of electrosurgical energy such that the tissue sealing surfaces are capable of conducting electrosurgical energy through tissue held therebetween to effect a seal, at least one of the first and second jaw members including an electrically conductive cutting element disposed thereon;
   a switch assembly operably disposed on the housing and including an activation member in operable communication with the source of electrosurgical energy for providing electrosurgical energy to the tissue sealing surfaces for sealing tissue; and
   a progressive touchpad operably disposed on the housing and in operable communication with at least one computer disposed in one of the housing and source of electrosurgical energy, the touchpad configured to provide electrosurgical energy to the cutting element for cutting the sealed tissue upon activation thereof.
13. An electrosurgical forceps, comprising:
   a housing supporting a rotatable shaft thereon;
   a pair of opposing first and second jaw members, each jaw member including a pair of spaced apart, electrically conductive tissue sealing surfaces adapted to connect to a source of electrosurgical energy such that the tissue sealing surfaces are capable of conducting electrosurgical energy through tissue held therebetween to effect a seal, at least one of the first and second jaw members including an electrically conductive cutting element disposed thereon; and
   a switch assembly operably disposed on the housing, the switch assembly including a magnetically-actuated activation member in operable communication with the source of electrosurgical energy for supplying electrosurgical energy to the first and second jaw members, the magnetically-actuated activation member including a first magnet configured to close corresponding first switch when brought into contact therewith and a second magnet configured to close a corresponding second switch when brought into contact therewith such that activation of the first switch provides electrosurgical energy to the tissue sealing surfaces for sealing tissue and activation of the second switch active provides electrosurgical energy to the cutting element for cutting the sealed tissue.
14. An electrosurgical forceps according to item 13, wherein the first and second magnets are supported in the housing and adjacent a proximal end of the shaft, and wherein movement of a slide on the housing brings the first and second magnets into contact with the corresponding switches.
15. An electrosurgical forceps, comprising:
   a housing;
   a pair of opposing first and second jaw members, at least one of the first and second jaw members being movable relative to the other from a first position wherein the first and second jaw members are disposed in spaced relation relative to one another to a second position wherein the first and second jaw members cooperate to grasp tissue therebetween, each jaw member including a pair of spaced apart, electrically conductive tissue sealing surfaces adapted to connect to a source of electrosurgical energy such that the tissue sealing surfaces are capable of conducting electrosurgical energy through tissue held therebetween to effect a seal, at least one of the first and second jaw members including an electrically conductive cutting element disposed thereon; and
   at least one sensor in operable communication with the source of electrosurgical energy, the at least one sensor configured to detect when the at least one moveable jaw member is in the second position and tissue is grasped between first and second jaw members such that with the first and second jaw members in the second position the source of electrosurgical energy automatically provides electrosurgical energy to the tissue sealing surfaces for sealing tissue and, subsequently, provides electrosurgical energy to the cutting element for cutting the sealed tissue.
16. An electrosurgical forceps according to item 15, wherein the at least one sensor is in operable communication with a movable handle of the electrosurgical forceps and is configured to detect when the movable handle has been moved into an unlatched configuration.
17. An electrosurgical forceps according to item 15 or 16, wherein the at least one sensor is in operable communication with the first and second jaw members and is configured to detect when the first and second jaw members are in the second position.

## Claims

1. An electrosurgical forceps, comprising:
a housing supporting a shaft thereon, the shaft defining a longitudinal axis therethrough;
a pair of opposing first and second jaw members, each jaw member including a pair of spaced apart, electrically conductive tissue sealing surfaces adapted to connect to a source of electrosurgical energy such that the tissue sealing surfaces are capable of conducting electrosurgical energy through tissue held therebetween to effect a seal, at least one of the first and second jaw members including an electrically conductive cutting element disposed thereon; and
a switch assembly operably disposed on the housing and including an activation member in operable communication with the source of electrosurgical energy for supplying electrosurgical energy to the first and second jaw members, the activation member movable from an inactive configuration to a first active configuration for providing electrosurgical energy to the tissue sealing surfaces for sealing tissue and a second active configuration for providing electrosurgical energy to the cutting element for cutting the sealed tissue.

2. An electrosurgical forceps according to claim 1, wherein activation member is operably disposed at a proximal end of the housing and is movable along the longitudinal axis to place the activation member in one of the first and second active configurations.

3. An electrosurgical forceps according to claim 1, wherein activation member is operably disposed adjacent a center of the housing and is movable across the longitudinal axis to place the activation member in one of the first and second active configurations.

4. An electrosurgical forceps according to claim 1 or 3, wherein the activation member is accessible from left or right sides of the housing.

5. An electrosurgical forceps according to claim 1, 2 or 3 wherein the activation member comprises one of a rocker switch and switch bar.

6. An electrosurgical forceps according to claim 5, wherein the rocker switch includes top and bottom portions that upon depression thereof are configured to place the rocker switch in respective first and second active configurations.

7. An electrosurgical forceps according to claim 5, wherein the switch bar includes top and bottom depressible portions that are configured to respectively place the switch bar in the first and second active configurations, the top and bottom depressible portions configured to illuminate when pressed.

8. An electrosurgical forceps according to claim 5 or 7, wherein the switch bar includes left and right depressible portions that are configured to adjust a power intensity level of the electrosurgical energy provided to the first and second jaw members, and optionally wherein a plurality of horizontally placed illuminable members are disposed between the left and right depressible portions to indicate to a user the power intensity level of the electrosurgical energy being provided to the first and second jaw members.
